# EUROPÄISCHE PATENTSCHRIFT

(11) **EP 0 475 184 B1**
(45) Veröffentlichungstag und Bekanntmachung des Hinweises auf die Patenterteilung: **08.02.1995**
(21) Anmeldenummer: 91114332.9
(22) Anmeldetag: 27.08.1991
(51) Int. Cl.: C07K 1/04, C07K 1/06

(54) **Verfahren zur Herstellung von Peptiden mittels Festphasensynthese**
Process for the production of peptides by solid phase synthesis
Procédé de production de peptides par synthèse à phase solide

(30) Priorität: 30.08.1990 DE 4027394
(43) Veröffentlichungstag der Anmeldung: 18.03.1992
(73) Patentinhaber: HOECHST AKTIENGESELLSCHAFT, 65926 Frankfurt am Main (DE)
(72) Erfinder: Breipohl, Gerhard, Dr., W-6000 Frankfurt am Main (DE); Knolle, Jochen, Dr., W-6239 Kriftel (DE); König, Wolfgang, Dr., W-6238 Hofheim a. Ts. (DE)

(56) Entgegenhaltungen:
- EP-A- 0 322 348

## Beschreibung

Die Erfindung betrifft ein Verfahren zur Herstellung von Peptiden mit C-terminalem Azaaminosäureamid mittels Festphasensynthese.

Der Erfindung liegt die Aufgabe zugrunde, ein racemisierungsarmes Verfahren zur Herstellung von Peptiden mit C-terminalen Azaaminosäureamiden mittels Festphasensynthese zu entwickeln.

Diese Aufgabe wird erfindungsgemäß gelöst durch das Verfahren zur Herstellung von Peptiden der allgemeinen Formel I

(X)ₙ - A - NH₂ (I),

in welcher
- X: eine natürliche oder unnatürliche Aminosäure, Azaaminosäure oder Iminosäure,
- n: eine ganze Zahl von 1 bis 50, bevorzugt 1 bis 30, und
- A: eine Azaaminosäure bedeuten,
sowie deren physiologisch verträgliche Salze, das dadurch gekennzeichnet ist, daß man einen Spacer in eine acylierungsfähige Form überführt, diesen mit einem geeigneten Ameisensäurederivat und anschließend mit einem entsprechenden Aminosäurehydrazid umsetzt, gegebenenfalls die Schutzgruppe in eine basenlabile oder gegen schwache Säuren labile Schutzgruppe überführt, den so erhaltenen Spacer an ein Harz kuppelt, das gewünschte Peptid stufenweise vom C-terminalen Ende aufbaut, anschließend das Peptid vom Harz abspaltet und gegebenenfalls in deren physiologisch verträgliche Salze überführt.

Natürliche oder unnatürliche Aminosäuren können, falls chiral, in der D- oder L-Form vorliegen. Bevorzugt sind α-Aminosäuren. Beispielsweise seien genannt:
Aad, Abu, γAbu, ABz, 2ABz, εAca, Ach, Acp, Adpd, Ahb, Aib, βAib, Ala, βAla, ΔAla, Alg, All, Ama, Amt, Ape, Apm, Apr, Arg, Asn, Asp, Asu, Aze, Azi, Bai, Bph, Can, Cit, Cys, (Cys)₂, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hlle, hLeu, hLys, hMet, hPhe, hPro, hSer, hThr, hTrp, hTyr, Hyl, Hyp, 3Hyp, Ile, Ise, Iva, Kyn, Lant, Lcn, Leu, Lsg, Lys, βLys, ΔLys, Met, Mim, Min, nArg, Nle, Nva, Oly, Orn, Pan, Pec, Pen, Phe, Phg, Pic, Pro, ΔPro, Pse, Pya, Pyr, Pza, Qin, Ros, Sar, Sec, Sem, Ser, Thi, βThi, Thr, Thy, Thx, Tia, Tle, Tly, Trp, Trta, Tyr, Val, Nal, Tbg, Npg, Cha, Chg, Thia (vgl. z.B. Houben-Weyl, Methoden der organischen Chemie, Band XV/1 und 2, Stuttgart, 1974).

Azaaminosäuren sind von natürlichen oder unnatürlichen Aminosäuren abgeleitet, wobei der Zentralbaustein -CHR- bzw. -CH₂- durch -NR- bzw. -NH- ersetzt ist. Beispielsweise seien Azaglycin, Azavalin, Azaleucin, Azaisoleucin und Azaphenylalanin genannt.

Unter einer Iminosäure werden allgemein natürliche oder unnatürliche Aminosäuren verstanden, deren Aminogruppe monosubstituiert ist. Besonders seien in diesem Zusammenhang Verbindungen erwähnt, die durch C₁-C₈-Alkyl, das wiederum gegebenenfalls einfach oder zweifach ungesättigt ist und das durch bis zu 3 gleiche oder verschiedene Reste aus der Reihe Mercapto; Hydroxy; C₁-C₇-Alkoxy; Carbamoyl; C₁-C₈-Alkanoyloxy; Carboxy; C₁-C₇-Alkoxycarbonyl; F; Cl; Br; I; Amino; Amidino, das gegebenenfalls durch einen, zwei oder drei C₁-C₈-Alkylreste substituiert sein kann; Guanidino, das gegebenenfalls durch einen oder zwei Benzyloxycarbonylreste oder durch einen, zwei, drei oder vier C₁-C₈-Alkylreste substituiert sein kann; C₁-C₇-Alkylamino; Di-C₁-C₇-Alkylamino; C₁-C₆-Alkoxycarbonylamino; C₇-C₁₅-Aralkoxycarbonyl; C₇-C₁₅-Aralkoxycarbonylamino; Phenyl-C₁-C₄-alkoxy; 9-Fluorenylmethoxycarbonylamino; C₁-C₆-Alkylsulfonyl; C₁-C₆-Alkylsulfinyl; C₁-C₆-Alkylthio; Hydroxyamino; Hydroxyimino; Sulfamoyl; substituiert sind.

Ferner kommen Heterocyclen aus der folgenden Gruppe in Betracht:
Pyrrolidin-2-carbonsäure; Piperidin-2-carbonsäure; 1,2,3,4-Tetrahydroisochinolin-3-carbonsäure; Decahydroisochinolin-3-carbonsäure; Octahydroindol-2-carbonsäure; Decahydrochinolin-2-carbonsäure; Octahydrocyclopenta[b]pyrrol-2-carbonsäure; 2-Aza-bicyclo[2.2.2]octan-3-carbonsäure; 2-Azabicyclo[2.2.1]heptan-3-carbonsäure; 2-Azabicyclo[3.1.0]hexan-3-carbonsäure; 2-Azaspiro[4.4]nonan-3-carbonsäure; 2-Azaspiro[4.5]decan-3-carbonsäure; Spiro[(bicyclo[2.2.1]heptan)-2,3-pyrrolidin-5-carbonsäure; Spiro[(bicyclo[2.2.2]octan)-2,3-pyrrolidin-5-carbonsäure; 2-Azatricyclo[4.3.0.1^{6,9}]decan-3-carbonsäure; Decahydrocyclohepta[b]pyrrol-2-carbonsäure; Octahydrocyclopenta[c]pyrrol-2-carbonsäure; Octahydroisoindol-1-carbonsäure; 2,3,3a,4,6a-Hexahydrocyclopenta[b]pyrrol-2-carbonsäure; 2,3,3a,4,5,7a-Hexahydroindol-2-carbonsäure; Tetrahydrothiazol-4-carbonsäure: Isoxazolidin-3-carbonsäure; Pyrazolidin-3-carbonsäure; Hydroxyprolin-2-carbonsäure; die alle gegebenenfalls substituiert sein können: 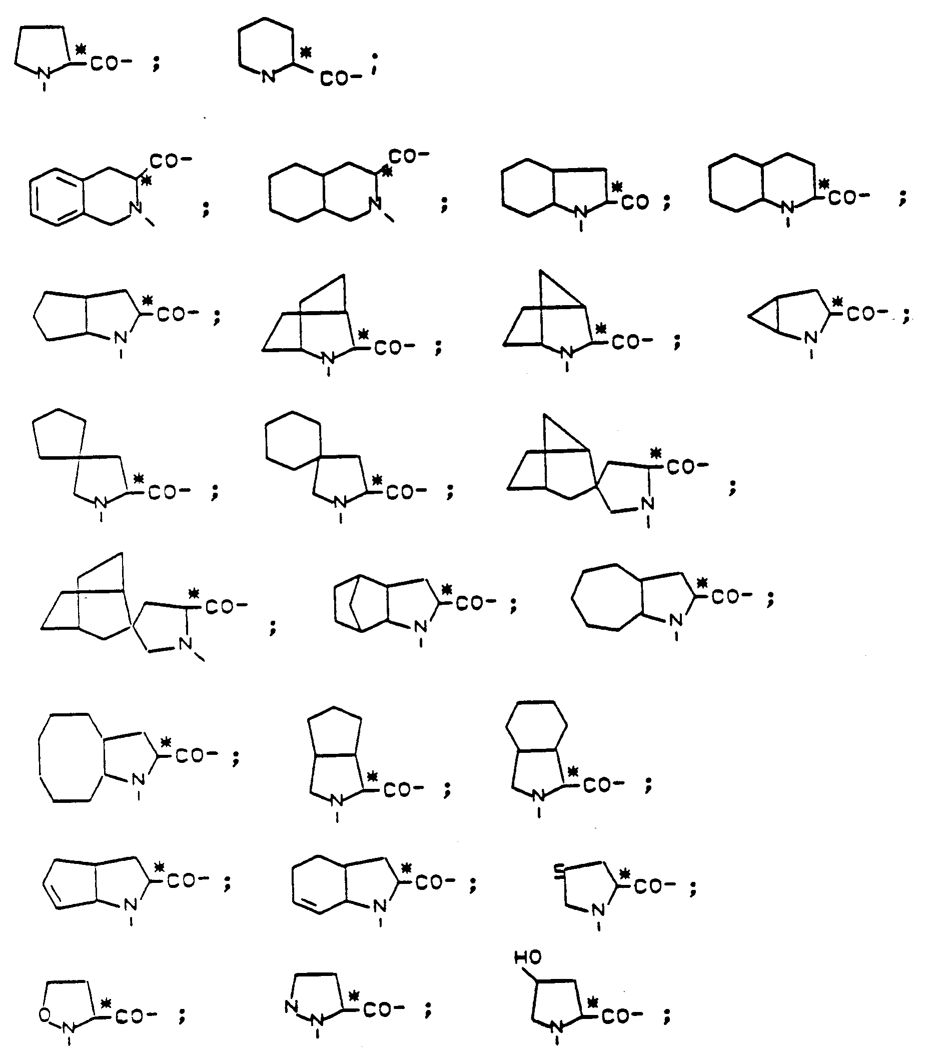
Unter Salzen von Verbindungen der Formel I sind insbesondere pharmazeutisch verwendbare oder nicht-toxische Salze zu verstehen. Insbesondere kommen Alkali- oder Erdalkalisalze, Salze mit physiologisch verträglichen Aminen und Salzen mit anorganischen oder organischen Säuren wie z.B. HCl, HBr, H₂SO₄, H₃PO₄, Maleinsäure, Fumarsäure, Citronensäure, Weinsäure, Essigsäure in Frage.

Man führt das Verfahren vorteilhaft in der Weise durch, daß man eine Verbindung der Formel II 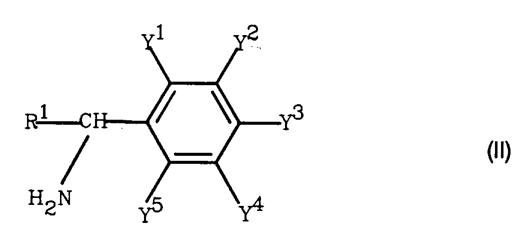
worin
- Y¹, Y², Y³, Y⁴ und Y⁵: Wasserstoff, C₁-C₄-Alkyl, C₁-C₄-Alkoxy oder -O-(CH₂)ₙ-COOH, -(CH₂)ₙ-COOH oder -NH-CO-(CH₂)ₙ-COOH, wobei die Reste gleich oder verschieden sein können, mindestens ein Rest jedoch für -O-(CH₂)ₙ-COOH, -(CH₂)ₙ-COOH oder -NH-CO-(CH₂)ₙ-COOH steht, n eine ganze Zahl von 1 bis 6, bevorzugt 1 bis 3, und
- R¹: Wasserstoff oder C₁-C₆-Alkoxy-C₆-C₁₂-aryl, bevorzugt 4-Methoxyphenyl bedeuten,
mit einem Silylierungsreagenz, beispielsweise tert. Butyldimethylsilylchlorid, tert. Butylphenylsilylchlorid, Trimethylchlorsilan, insbesondere Trimethylchlorsilan, in einem dafür geeigneten Lösungsmittel, wie z.B. THF, Acetonitril, Methylenchlorid, Dimethylformamid oder Mischungen derselben umsetzt und anschließend die silylierte Verbindung mit einem Chlorameisensäurederivat, insbesondere den substituierten Esterderivaten, in Verbindungen der Formel III überführt, 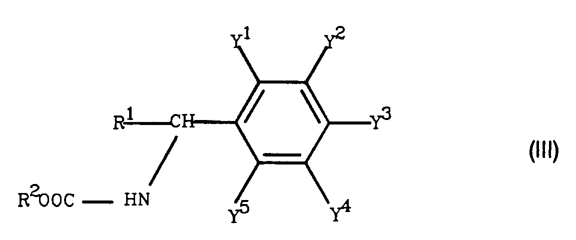
worin
- R¹, Y¹, Y², Y³, Y⁴ und Y⁵: wie oben definiert sind und
- R²: ein C₆-C₁₂-Aryl-Rest bedeutet, welcher durch elektronenanziehende Substituenten, vorzugsweise Nitro und Halogen, beispielsweise F oder Cl, substituiert ist,
die so erhaltenen Verbindungen der Formel III mit einem Aminosäurehydrazid der Formel IV

R³ - X - CO - NH - NH - R⁴ (IV)

worin
- X: eine natürliche oder unnatürliche Aminosäure oder Iminosäure bedeutet und wie oben definiert ist,
- R³: für eine basenlabile oder gegen schwache Säuren bzw. Hydrierung labile Schutzgruppe, wie z.B. eine Urethanschutzgruppe (siehe z.B. Hubbuch, Kontakte (Merck) 1979, Nr. 3, Seiten 14 bis 23) steht und
- R⁴: C₁-C₈-Alkyl, C₃-C₉-Cycloalkyl, C₆-C₁₂-Aryl, C₆-C₁₂-Aryl-C₁-C₈-alkyl, Heteroaryl oder Heteroaryl-C₁-C₈-alkyl oder Wasserstoff bedeutet,
in einem Lösungsmittel, in dem die Verbindungen der Formel III und IV löslich sind, wie z.B. DMF, zu den Verbindungen der Formel V umsetzt, 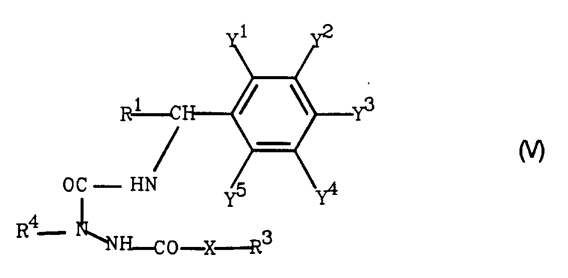
worin R¹, R³, R⁴ und Y¹, Y², Y³, Y⁴ und Y⁵ die oben angegebenen Bedeutungen haben, falls R³ für eine gegen Hydrierung labile Schutzgruppe steht, vorzugsweise Benzyloxycarbonyl, diese Schutzgruppe durch Hydrierung über einem Pd-Katalysator entfernt und vor der weiteren Reaktion in eine basenlabile Urethanschutzgruppe, vorzugsweise Fmoc, oder eine gegen schwache Säuren labile Urethanschutzgruppe, vorzugsweise Bpoc, überführt,
anschließend die Verbindung der Formel V, worin R¹, R⁴, Y¹, Y², Y³, Y⁴ und Y⁵ die oben angegebenen Bedeutungen haben und R³ eine gegen Basen bzw. schwache Säuren labile Urethanschutzgruppe bedeutet, mit den in der Peptidchemie üblichen Kupplungsreagenzien über die -O-(CH₂)ₙ-COOH, -(CH₂)ₙ-COOH oder -NH-CO-(CH₂)ₙ-COOH Gruppierung an ein Harz kuppelt, die Schutzgruppe R³ abspaltet, stufenweise durch basenlabile oder gegen schwache Säuren labile Aminoschutzgruppen temporär geschützte natürliche oder unnatürliche Amino-, Imino- oder Azaaminosäuren, gegebenenfalls in Form ihrer aktivierten Derviate, ankuppelt und nach beendetem Aufbau die Peptide der Formel I durch Behandeln mit einer mittelstarken Säure vom Harz freisetzt, wobei gleichzeitig oder durch geeignete Maßnahmen daran anschließend temporär eingeführte Seitenkettenschutzgruppen wieder abgespalten werden.

Die Verbindungen der Formel IV stellt man durch Umsetzung der natürlichen oder unnatürlichen Aminosäuren bzw. Iminosäure mit den entsprechenden Hydrazinen nach den in der Peptidchemie üblichen Kupplungsmethoden her.

Alkyl kann geradkettig oder verzweigt sein. Entsprechendes gilt für davon abgeleitete Reste, wie z.B. Alkoxy, Alkylthio, Alkylamino, Dialkylamino und Alkanoyl. Insbesondere steht Alkyl für C₁-C₄-Alkyl.

Unter Cycloalkyl werden auch alkylsubstituierte Reste, wie z.B. 4-Methylcyclohexyl oder 2,3-Dimethylcyclopentyl verstanden.

Halogen steht für Fluor, Chlor, Brom oder Jod, insbesondere für Fluor oder Chlor.

C₆-C₁₂-Aryl ist beispielsweise Phenyl oder Naphthyl, bevorzugt ist Phenyl. Heteroaryl steht für den Rest eines 5- bis 7-gliedrigen monocyclischen oder 8- bis 10-gliedrigen bicyclischen aromatischen Ringsystems, das benzanelliert sein kann, das als Heteroelemente einen, zwei, drei oder vier verschiedene Reste aus der Gruppe N, O, S, NO, SO, SO₂ enthalten kann, das mit 1 bis 6 Hydroxy oder durch einen, zwei oder drei gleiche oder verschiedene Reste aus der Reihe F, Cl, Br, I; Hydroxy; Mono-, Di- oder Trihydroxy-C₁-C₄-alkyl; Trifluormethyl; Formyl; Carboxamido; Mono- oder Di-C₁-C₄-alkylaminocarbonyl; Nitro; C₁-C₇-Alkoxy; C₁-C₇-Alkyl; C₁-C₇-Alkoxycarbonyl, Amino; C₁-C₇-Alkylamino; Di-C₁-C₇-Alkylamino; Carboxy; Carboxymethoxy; Amino-C₁-C₇-alkyl; C₁-C₇-Alkylamino-C₁-C₇-alkyl; Di-C₁-C₇-alkylamino-C₁-C₇-alkyl; C₁-C₇-Alkoxycarbonylmethoxy; Carbamoyl; Sulfamoyl; C₁-C₇-Alkoxysulfonyl; C₁-C₈-Alkylsulfonyl; Sulfo-C₁-C₈-alkyl; Guanidino-C₁-C₈-alkyl und C₁-C₆-Alkoxycarbonyl und/oder mit Oxo, mono-, di oder trisubstituiert ist. Insbesondere seien genannt: Furyl, Thienyl, Imidazolyl, Pyrazolyl, Oxazolyl, Isoxazolyl, Thiazolyl, Isothiazolyl, Tetrazolyl, Pyridyl, Pyrazinyl, Pyrimidinyl, Indolyl, Isoindolyl, Indazolyl, Phthalazinyl, Chinolyl, Isochinolyl, Chinoxalinyl, Chinazolinyl, Cinnolinyl, β-Carbolinyl, oder ein benzanelliertes Derivat dieser Reste.

Falls zur Verhinderung von Nebenreaktionen oder für die Synthese spezieller Peptide erforderlich, sind die funktionellen Gruppen in der Seitenkette von Amino-, Azaamino- bzw. Iminosäuren durch geeignete Schutzgruppen (siehe z.B. T.W. Greene, "Protective Groups in Organic Synthesis", New York, John Wiley & Sons, 1981; Hubbuch, Kontakte (Merck) 1979, Nr. 3, Seiten 14-23; Büllesbach, Kontakte (Merck) 1980, Nr. 1, Seiten 23-35) zusätzlich geschützt, wobei in erster Linie Arg(Tos), Arg(Mts), Arg(Mtr), Arg(Pmc), Asp(OBzl), Asp (OtBu), Cys(4-MeBzl), Cys(Acm), Cys(StBu), Glu(OBzl), Glu(OtBu), His(Tos), His(Fmoc), His(Dnp), His(Trt), Lys(Cl-2), Lys(Boc), Met(O), Ser(Bzl), Ser(tBu), Thr(Bzl), Thr(tBu) eingesetzt werden. Ferner können die funktionellen Gruppen in der Seitenkette auch glycosyliert sein, wie z.B. in EP-A 263 521 (HOE 86/F 253) beschrieben.

Die als Trägermaterial verwendeten Harte sind kommerziell erhältlich oder selbst hergestellt, wie z.B. Alkoxybenzylalkoholharze, Aminomethylharze oder Benzhydrylaminoharze. Bevorzugt sind Aminomethyl-, Benzhydrylamino- (BHA) und Methylbenzhydrylamino-Harze (MBHA). Die Beladung bestimmt man durch Aminosäureanalyse und/oder Elementaranalyse.

Unter geeigneten Spacern sind beispielsweise die in Atherton, Sheppard in Perspectives in Peptide Chemistry, Seiten 101-117 (Karger, Basel 1981); EP-A 264 802 (HOE 86/F 259), EP-A 287 882 (HOE 87/F 101) und EP-A 322 348 (HOE 87/F 386K) beschriebenen Spacer sowie davon abgeleitete Derivate zu verstehen, wie beispielsweise solche, deren Schutzgruppe abgespalten ist. Bevorzugt sind 4-Carboxylatopropoxy-4'-methoxy-benzhydrylamin und 5-Carboxylatoethyl-2,4-dimethoxy-4'-methoxy-benzhydrylamin.

Als Kupplungsreagenz für den Spacer der Formel V und die weiteren Aminosäurederivate können alle möglichen in der Peptidsynthese verwendeten Aktivierungs-Reagenzien, siehe z.B. Houben-Weyl, Methoden der Organischen Chemie, Band XV/2, Stuttgart 1974 verwendet werden, insbesondere aber Carbodiimide wie z.B. N,N'-Dicyclohexylcarbodiimid, N,N'-Diisopropylcarbodiimid oder N-Ethyl-N'-(3-dimethylaminopropyl)carbodiimid. Die Kupplung kann dabei direkt durch Addition von Aminosäurederivat mit dem Aktivierungsreagenz und gegebenenfalls einem die Racemisierung unterdrückenden Zusatz wie z.B. 4-Dimethylaminopyridin, 1 - Hydroxybenzotriazol (HOBt) (W. König, R. Geiger, Chem. Ber. 103 (1970) 788 - 798) oder 3-Hydroxy-4-oxo-3,4-dihydrobenzotriazin (HOObt) (W. König, R. Geiger, Chem. Ber. 103 (1970) 2034 - 2040) zum Harz durchgeführt werden oder aber die Voraktivierung des Aminosäurederivats als symmetrisches Anhydrid oder HOBt- bzw. HOObt-Ester kann separat erfolgen und die Lösung der aktivierten Spezies in einem geeigneten Lösungsmittel zum kupplungsfähigen Peptidharz gegeben werden.

Die Kupplung bzw. Aktivierung des Spacers der Formel V und der Aminosäurederivate mit einem der obengenannten Aktivierungsreagenzien kann in Dimethylformamid oder Methylenchlorid oder einer Mischung aus beiden durchgeführt werden. Das aktivierte Aminosäurederivat wird üblicherweise in einem 1,5- bis 4-fachen Überschuß eingesetzt. In Fällen, in denen eine unvollständige Kupplung eintritt, wird die Kupplungsreaktion wiederholt, ohne vorher die für die Kupplung der nächstfolgenden Aminosäure nötige Entblockung der α-Aminogruppe des Peptidharzes durchzuführen.

Der erfolgreiche Ablauf der Kupplungsreaktion kann mittels der Ninhydrin-Reaktion, wie z.B. von E. Kaiser et al. (Anal. Biochem. 34 (1970) 595) beschrieben, überprüft werden. Die Synthese kann auch automatisiert z.B. mit einem Peptid-Synthesizer Model 430A der Fa. Applied Biosystems durchgeführt werden, wobei entweder die vom Gerätehersteller vorgesehenen Syntheseprogramme oder aber auch vom Benutzer selbst erstellte verwendet werden können. Letztere werden insbesondere bei der Verwendung von mit der Fmoc-Gruppe geschützten Aminosäurederivaten eingesetzt.

Die Abspaltung der Peptidamide vom Harz erfolgt durch Behandlung mit in der Peptidsynthese üblicherweise verwendeten mittelstarken Säuren (z.B. Trifluoressigsäure) wobei als Kationenfänger Substanzen wie Phenol, Kresol, Thiokresol, Anisol, Thioanisol, Ethandithiol, Dimethylsulfid, Ethylmethylsulfid oder ähnliche in der Festphasensynthese übliche Kationenfänger einzeln oder eine Mischung von zwei oder mehr dieser Hilfsmittel zugesetzt werden. Die Trifluoressigsäure kann dabei auch durch geeignete Lösungsmittel, wie z.B. Methylenchlorid, verdünnt angewendet werden.
Bei der Abspaltung des Spacers vom Harz erfolgt gleichzeitig die Abspaltung der Seitenkettenschutzgruppen.

Die Reinigung der so erhaltenen Rohpeptide erfolgt mittels Chromatographie an ®Sephadex, Ionenaustauscherharzen oder HPLC.

Bevorzugt ist ein Verfahren der Festphasensynthese zur Herstellung von Ac-D-Nal(2)-p-Cl-D-Phe-D-Trp-Ser-Tyr-D-Ser(α-L-Rha)-Leu-Arg-Pro-Azagly-NH₂ und pGlu-His-Trp-Ser-Tyr-D-Ser(tBu)-Leu-Arg-Pro-Azagly-NH₂ (Zoladex).

Verzeichnis der verwendeten Abkürzungen:
- BSA: Bistrimethylsilylacetamid
- Cha: Cyclohexylalanin
- Chg: Cyclohexylglycin
- DCC: Dicyclohexycarbodiimid
- DIC: Diisopropylcarbodiimid
- DMAP: Dimethylaminopyridin
- Fmoc: 9-Fluorenylmethoxycarbonyl
- HOBt: 1-Hydroxybenzotriazol
- HOObt: 3-Hydroxy-4-oxo-3,4-dihydro-1,2,3-benzotriazin
- Nal: Naphthylalanin
- Npg: Neopentylglycin
- Pmc: 2,2,5,7,8-Pentamethylchroman-6-sulfonyl
- Tbg: Tert.-butylglycin
- THF: Tetrahydrofuran
- Thia: 2-Thienylalanin
Die nachstehenden Beispiele dienen zur Erläuterung der vorstehenden Erfindung, ohne daß sie darauf beschränkt wäre.

### Beispiel 1

### a) 5-Carboxylatoethyl-2,4-dimethoxy-4'-methoxy-benzhydrylamin

17,5 g 5-Carboxylatoethyl-2,4-dimethoxy-4'-methoxy-benzophenonoxim wurden in 450 ml einer 1:1 Mischung aus Ethanol und DMF gelöst und mit 2 ml konz. NH₃ versetzt. Nach Zugabe des Pt/C-Katalysators wurde bei Normaldruck für 5 Tage hydriert. Nach Beendigung der Reaktion wurde der Katalysator abgesaugt, das Filtrat eingeengt und das Produkt mit Ether ausgefällt. Es wurde als solches ohne weitere Reinigung eingesetzt.

### 1b) N-(p-Nitrophenyloxycarbonyl)-5-carboxylatoethyl-2,4-dimethoxy-4'-methoxy-benzhydrylamin

10 g der Titelverbindung aus Beispiel 1a) wurden in 100 ml einer THF/DMF 4:1 Mischung vorgelegt und bei Raumtemperatur mit 2,1 equiv. Bistrimethylsilylacetamid (BSA) versetzt. Die Suspension klärte sich in kurzer Zeit vollständig und die klare Lösung wurde für 2 Stunden nachgerührt. Dann wurden 3 g Chlorameisensäurenitrophenylester zugesetzt und eine weitere Stunde nachgerührt. Nach Beendigung der Reaktion wurde das Lösungsmittel im Hochvakuum entfernt. Der Rückstand wird mit 300 ml Wasser versetzt und das entstehende Öl mit Essigester extrahiert. Die Essigester-Phase wurde mit einer 1 N KHSO₄-Lösung und Wasser gewaschen. Die organische Phase wurde über MgSO₄ getrocknet und zur Trockene eingedampft. Der Rückstand (12 g) wird durch NMR, IR und MS charakterisiert.

N-(p-Nitrophenyloxycarbonyl)-5-carboxylatoethyl-2,4-dimethoxy-4'-methoxy-benzhydrylamin wurde anschließend mit Aminosäurehydraziden zu den geeigneten substituierten Ankern umgesetzt.

### 1c) Benzyloxycarbonyl-4-prolyl-azaglycin-(5-carboxylatoethyl-2,4-dimethoxy-4'-methoxybenzhydryl)amid.

3,27 g Benzyloxycarbonyl-prolylhydrazid Hydrochlorid und 6,94 g der Titelverbindung aus Beispiel 1b) wurden in 40 ml Dimethylformamid (DMF) gelöst und mit 3 equiv. N-Ethylmorpholin und einer katalytischen Menge von Dimethylaminopyridin (DMAP) versetzt. Man ließ 16 Stunden reagieren. Nach Beendigung der Reaktion wurde zur Trockene eingedampft. Der Rückstand wurde in Essigester/Butanol aufgenommen und die organische Phase mit gesättigter NaHCO₃-Lösung, 1 N KHSO₄-Lösung und Wasser gewaschen. Die organische Phase wurde über MgSO₄ getrocknet und nach Filtration zur Trockene eingedampft. Der Rückstand ließ sich aus reinem Essigester umkristallisieren. Man erhielt 6,6 g der Titelverbindung.
FAB-MS: 641 (M+Li⁺)
IR: CO 1695 cm⁻¹
¹H-NMR (DMSO): δ = 3,7 s (6H, OCH₃) ppm

### 1d) 9-Fluorenylmethoxycarbonyl-L-prolyl-azaglycin(5-carboxylatoethyl-2,4-dimethoxy-4'-methoxy-benzhydryl)amid

26,5 g der Titelverbindung aus Beispiel 1c) wurden in 300 ml Methanol gelöst und mit 2 g Pd/C-Katalysator versetzt. Nach einer Stunde war die Hydrierung beendet. Der Katalysator wurde abfiltriert und das Filtrat zur Trockene eingeengt. Ohne weitere Reinigung wurde der Rückstand (17,5 g) in einer Mischung aus 80 ml Wasser und 80 ml Dioxan aufgenommen und mit 8 g Natriumhydrogencarbonat und 17 g N-(9-Fluorenylmethoxycarbonyloxy)succinimid (Fmoc-ONSu) versetzt. Man ließ einen Tag reagieren. Nach Beendigung der Reaktion wurde über Klärschichtfilter filtriert. Das Filtrat wurde mit 2 N H₂SO₄ auf pH 6 gestellt und im Vakuum auf ein Volumen von 80 ml eingedampft. Man verdünnte mit 100 ml Wasser und extrahierte mit einer Mischung aus Essigester/n-Butanol (8,5:1,5). Die organische Phase wurde mit halbgesättigter NaCl-Lösung gewaschen und dann zur Trockene eingedampft. Der Rückstand wurde über 500 g Silicagel mit Essigester filtriert. Man erhielt 20 g der Titelverbindung.
FAB-MS: 729 (M+Li⁺)
IR: CO 1695 cm⁻¹

### 1e) Kupplung der Titelverbindung aus Beispiel 1d) an ein Polystyrolharz

1,0 g Aminomethylpolystyrolharz (Beladung 1,07 mmol) und 1,2 g der Titelverbindung aus Beispiel 1d) wurden in 10 ml Dimethylformamid suspendiert und mit 216 mg 1-Hydroxy-benzotriazol (HOBt) und 0,75 ml Diisopropylcarbodiimid (DIC) versetzt. Man ließ über Nacht reagieren bis der Ninhydrin-Test eine vollständige Reaktion anzeigte. Das Harz wurde abfiltriert und mit Dimethylformamid und Methylenchlorid gewaschen und gründlich im Vakuum getrocknet. Die Beladung des Harzes mit Prolin betrug 0,51 mmol/g.

### 1f) Ac-D-Nal(2)-p-Cl-D-Phe-D-Trp-Ser-Tyr-D-Ser(α-L-Rha)-Leu-Arg-Pro-Azagly-NH₂

Die 9-Fluorenylmethoxycarbonyl-N_{α}-aminoschutzgruppe der Verbindung aus Beispiel 1e) wurde mit einer 20 %igen Piperidin/Dimethylformamid-Lösung abgespalten (2x3 min, 2x8 ml). Anschließend wurde das Hart mit N-Methylpyrrolidinon (5x10 ml) nachgewaschen und das Peptid am Harz (785 mg Harz aus Beispiel 1c)) aufgebaut, wobei folgende Schritte cyclisch durchlaufen wurden:
- Abspaltung der Fmoc-Schutzgruppe mit 20 % Piperidin in DMF
- Waschen des Harzes mit DMF / N-Methylpyrrolidinon
- Aufkuppeln der Fmoc-Aminosäure unter in situ Aktivierung als HOBt-Ester unter Verwendung von Diisopropylcarbodiimid als Aktivierungsreagenz (1,5 mmol Aminosäure, 2,25 mmol HOBt, 1,6 mmol Diisopropylcarbodiimid)
Falls die Kupplung unvollständig war (Kaiser-Test) wurde der Kupplungsschritt wiederholt. Als letzte Aminosäure wurde Fmoc-D-Nal(2)-OH eingesetzt. Die N-terminale Acetylgruppe wurde durch Reaktion mit Acetanhydrid eingeführt.

Nach Beendigung der Festphasensynthese wurde das Harz gewaschen (DMF, CH₂Cl₂) und gründlich getrocknet. Man erhieit 1,35 g substituiertes Harz.

Das getrocknete Harz wurde bei Raumtemperatur mit 0,75 ml Ethandithiol suspendiert. Nach 15 Minuten wurden 7,5 ml Trifluoressigsäure hinzugefügt und die Suspension für 1,5 Stunden gerührt. Nach dieser Zeit wurde das Hart abfiltriert und gründlich mit 80 %iger Trifluoressigsäure gewaschen. Das Filtrat wurde im Vakuum eingedampft und in 30 ml Wasser aufgenommen. Durch Zugabe von NaHCO₃ wurde pH 6-7 eingestellt und das Peptid mit n-Pentanol ausgeschüttelt (4x30 ml). Die n-Pentanolphase wurde eingedampft und in 10 ml Methanol/H₂O (9:1) aufgenommen und mit 0,5 g K₂CO₃ versetzt. Man rührte 30 Minuten, filtrierte und engte das Filtrat ein. Der Rückstand wurde in 100 ml n-Pentanol aufgenommen und die organische Phase mit Wasser gewaschen. Nach Trocknen mit MgSO₄ und Filtration wurde die organische Phase eingedampft. Man erhielt 740 mg an Rohprodukt Nach Chromatographie an ®Sephadex G 25(1 M Essigsäure) und an Silicagel erhielt man 185 mg der reinen Titelverbindung.
FAB-MS: 1531 (M+H⁺)

## Patentansprüche

1. Verfahren zur Herstellung von Peptiden der allgemeinen Formel I
(X)ₙ - A - NH₂ (I),
in welcher
X eine natürliche oder unnatürliche Aminosäure, Azaaminosäure oder Iminosäure,
n eine ganze Zahl von 1 bis 50, bevorzugt 1 bis 30, und
A eine Azaaminosäure bedeuten,
sowie deren physiologisch verträgliche Salze, das dadurch gekennzeichnet ist, daß man einen Spacer in eine acylierungsfähige Form überführt, diesen mit einem geeigneten Ameisensäurederivat und anschließend mit einem entsprechenden Aminosäurehydrazid umsetzt, gegebenenfalls die Schutzgruppe in eine basenlabile oder gegen schwache Säuren labile Schutzgruppe überführt, den so erhaltenen Spacer an ein Harz kuppelt, das gewünschte Peptid stufenweise vom C-terminalen Ende aufbaut, anschließend das Peptid vom Harz abspaltet und gegebenenfalls in deren physiologisch verträgliche Salze überführt.

2. Verfahren gemäß Anspruch 1, dadurch gekennzeichnet, daß man eine Verbindung der Formel II 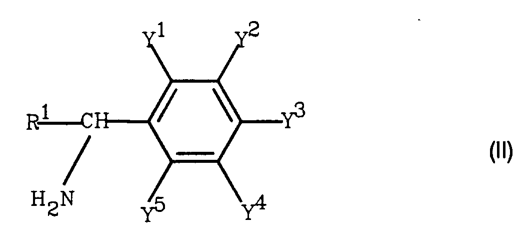 worin
Y¹, Y², Y³, Y⁴ und Y⁵ Wasserstoff, C₁-C₄-Alkyl, C₁-C₄-Alkoxy oder -O-(CH₂)ₙ-COOH, -(CH₂)ₙ-COOH oder -NH-CO-(CH₂)ₙ-COOH, wobei die Reste gleich oder verschieden sein können, mindestens ein Rest jedoch für -O-(CH₂)ₙ-COOH, -(CH₂)ₙ-COOH oder -NH-CO-(CH₂)ₙ-COOH steht, n eine ganze Zahl von 1 bis 6, und
R¹ Wasserstoff oder C₁-C₆-Alkoxy-C₆-C₁₂-aryl bedeuten,
mit einem Silylierungsreagenz in einem dafür geeigneten Lösungsmittel umsetzt und anschließend die silylierte Verbindung mit einem Chlorameisensäurederivat in Verbindungen der Formel III überführt, 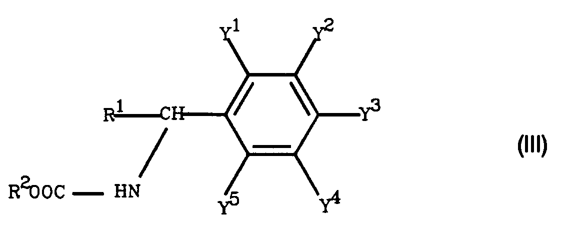 worin
R¹, Y¹, Y², Y³, Y⁴ und Y⁵ wie oben definiert sind und
R² ein C₆-C₁₂-Aryl-Rest bedeutet, welcher durch elektronenanziehende Substituenten, vorzugsweise Nitro und Halogen substituiert ist,
die so erhaltenen Verbindungen der Formel III mit einem Aminosäurehydrazid der Formel IV
R³ - X - CO - NH - NH - R⁴ (IV)
worin
X eine natürliche oder unnatürliche Aminosäure oder Iminosäure bedeutet und wie oben definiert ist,
R³ für eine basenlabile oder gegen schwache Säuren bzw. Hydrierung labile Schutzgruppe steht und
R⁴ C₁-C₈-Alkyl, C₃-C₉-Cycloalkyl, C₆-C₁₂-Aryl, C₆-C₁₂-Aryl-C₁-C₈-alkyl, Heteroaryl oder Heteroaryl-C₁-C₈-alkyl oder Wasserstoff bedeutet,
in einem geeigneten Lösungsmittel zu den Verbindungen der Formel V umsetzt, 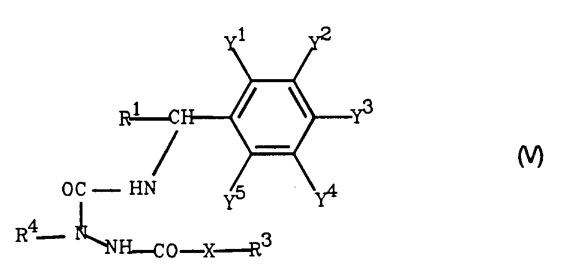 worin R¹, R³, R⁴ und Y¹, Y², Y³, Y⁴ und Y⁵ die oben angegebenen Bedeutungen haben, falls R³ für eine gegen Hydrierung labile Schutzgruppe steht, diese Schutzgruppe durch Hydrierung über einem Pd-Katalysator entfernt und vor der weiteren Reaktion in eine basenlabile oder eine gegen schwache Säuren labile Urethanschutzgruppe überführt, anschließend die Verbindung der Formel V, worin R¹, R⁴, Y¹, Y², Y³, Y⁴ und Y⁵ die oben angegebenen Bedeutungen haben und R³ eine gegen Basen bzw. schwache Säuren labile Urethanschutzgruppe bedeutet, mit den in der Peptidchemie üblichen Kupplungsreagenzien über die -O-(CH₂)ₙ-COOH, -(CH₂)ₙ-COOH oder -NH-CO-(CH₂)ₙ-COOH Gruppierung an ein Harz kuppelt, die Schutzgruppe R³ abspaltet, stufenweise durch basenlabile oder gegen schwache Säuren labile Aminoschutzgruppen temporär geschützte natürliche oder unnatürliche Amino-, Imino- oder Azaaminosäuren, gegebenenfalls in Form ihrer aktivierten Derviate, ankuppelt und nach beendetem Aufbau die Peptide der Formel I durch Behandeln mit einer mittelstarken Säure vom Harz freisetzt, wobei gleichzeitig oder durch geeignete Maßnahmen daran anschließend temporär eingeführte Seitenkettenschutzgruppen wieder abgespalten werden.

3. Verfahren gemäß einem der Ansprüche 1 bis 2, dadurch gekennzeichnet, daß
Ac-D-Nal(2)-p-Cl-D-Phe-D-Trp-Ser-Tyr-D-Ser(α-L-Rha)-Leu-Arg-Pro-Azagly-NH₂
hergestellt wird.

4. Verfahren gemäß einem der Ansprüche 1 bis 2, dadurch gekennzeichnet, daß
pGlu-His-Trp-Ser-Tyr-D-Ser(tBu)-Leu-Arg-Pro-Azagly-NH₂
hergestellt wird.

## Claims

1. A process for the preparation of peptides of the formula I
(X)ₙ-A-NH₂ (I)
in which
X is a natural or unnatural amino acid, aza-amino acid or imino acid,
n is an integer from 1 to 50, preferably 1 to 30, and
A is an aza-amino acid,
and the physiologically tolerated salts thereof, which comprises converting a spacer into a form capable of acylation, reacting the latter with a suitable formic acid derivative and subsequently with an appropriate amino acid hydrazide, where appropriate converting the protective group into a protective group which is base-labile or labile to weak acids, coupling the spacer obtained in this way to a resin, synthesizing the required peptide stepwise from the C-terminal end, subsequently cleaving the peptide off the resin and, where appropriate, converting it into physiologically tolerated salts thereof.

2. The process as claimed in claim 1, wherein a compound of the formula II 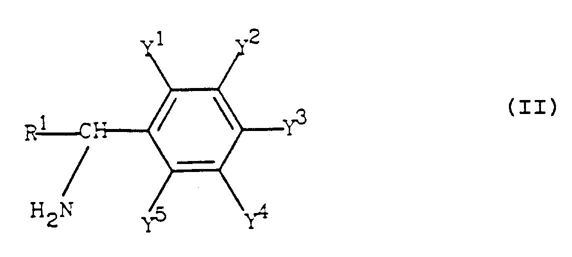 in which
Y¹, Y², Y³, Y⁴ and Y⁵ are hydrogen, C₁-C₄-alkyl, C₁-C₄-alkoxy or -O-(CH₂)ₙ-COOH, -(CH₂)ₙ-COOH or -NH-CO-(CH₂)ₙ-COOH, where the radicals can be identical or different, but at least one radical is -O-(CH₂)ₙ-COOH, -(CH₂)ₙ-COOH or -NH-CO-(CH₂)ₙ-COOH, n is an integer from 1 to 6, and
R¹ is hydrogen or C₁-C₆-alkoxy-C₆-C₁₂-aryl,
is reacted with a silylating reagent in a solvent suitable for this purpose, and subsequently the silylated compound is converted with a chloroformic acid derivative into compounds of the formula III 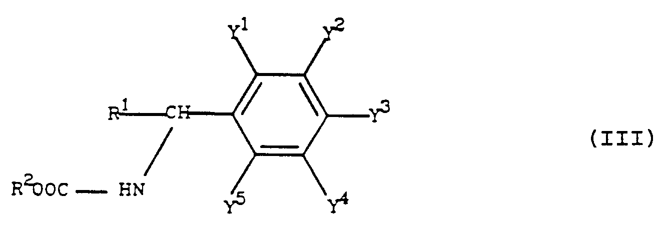 in which
R¹, Y¹, Y², Y³, Y⁴ and Y⁵ are as defined above, and
R² is a C₆-C₁₂-aryl radical which is substituted by electron-attracting substituents, preferably nitro and halogen,
the compounds of the formula III obtained in this way are reacted with an amino acid hydrazide of the formula IV
R³-X-CO-NH-NH-R⁴ (IV)
in which
X is a natural or unnatural amino acid or imino acid and is as defined above,
R³ is a protective group which is base-labile or labile to weak acids or hydrogenation, and
R⁴ is C₁-C₈-alkyl, C₃-C₉-cycloalkyl, C₆-C₁₂-aryl, C₆-C₁₂-aryl-C₁-C₈-alkyl, heteroaryl or heteroaryl-C₁-C₈-alkyl or hydrogen,
in a suitable solvent to give the compounds of the formula V 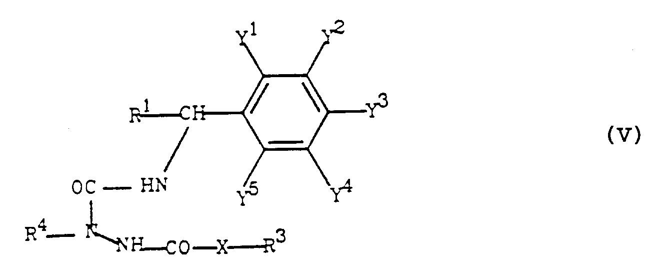 in which R¹, R³, R⁴ and Y¹, Y², Y³, Y⁴ and Y⁵ have the abovementioned meanings, if R³ is a protective group labile to hydrogenation this protective group is removed by hydrogenation on a Pd catalyst and, before the subsequent reaction, converted into a urethane protective group which is base-labile or labile to weak acids, subsequently the compound of the formula V in which R¹, R⁴ Y¹, Y², Y³, Y⁴ and Y⁵ have the abovementioned meanings, and R³ is a urethane protective group which is labile to bases and weak acids, is coupled with the coupling reagents customary in peptide chemistry via the -O-(CH₂)ₙ-COOH, -(CH₂)ₙ-COOH or -NH-CO-(CH₂)ₙ-COOH group to a resin, the protective group R³ is eliminated, natural or unnatural amino, imino or aza-amino acids which have been temporarily protected by amino-protective groups which are base-labile or labile to weak acids and which are optionally in the form of their activated derivatives are coupled on stepwise, and, after the synthesis is complete, the peptides of the formula I are liberated from the resin by treatment with a moderately strong acid, with elimination again, simultaneously or by suitable measures subsequent thereto, of temporarily introduced side-chain protective groups.

3. The process as claimed in either of claims 1 and 2, wherein Ac-D-Nal(2)-p-Cl-D-Phe-D-Trp-Ser-Tyr-D-Ser(α-L-Rha)-Leu-Arg-Pro-Azagly-NH₂ is prepared.

4. The process as claimed in either of claims 1 and 2, wherein pGlu-His-Trp-Ser-Tyr-D-Ser(tBu)-Leu-Arg-Pro-Azagly-NH₂ is prepared.

## Revendications

1. Procédé pour la préparation de peptides de formule générale I
(X)ₙ-A-NH₂ (I)
dans laquelle
X représente un aminoacide, aza-aminoacide ou iminoacide naturel ou non,
représente un nombre entier de 1 à 50, de
préférence de 1 à 30, et
A représente un aza-aminoacide,
ainsi que de leurs sels physiologiquement acceptables, caractérisé en ce que l'on convertit un espaceur en une forme apte à l/acylation, on le fait réagir avec un dérivé d'acide formique convenable et ensuite avec un hydrazide d'aminoacide approprié, éventuellement on convertit le groupe protecteur en un groupe protecteur labile en présence de bases ou labile en présence d'acides faibles, l'espaceur ainsi obtenu est couplé à une résine, le peptide recherché est synthétisé graduellement à partir de l'extrémité C-terminale, puis le peptide est séparé de la résine et éventuellement converti en ses sels physiologiquement acceptables.

2. Procédé selon la revendication 1, caractérisé en ce que l'on fait réagir composé de formule II 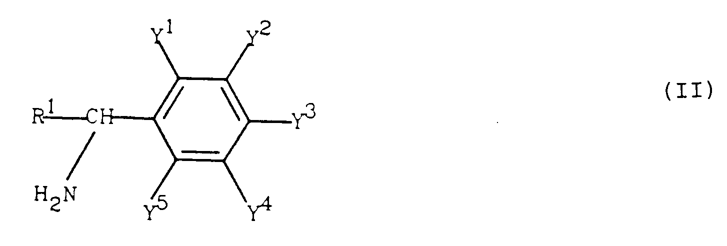 dans laquelle
Y¹, Y², Y³, Y⁴ et Y⁵ représentent un atome d/hydrogène ou un radical alkyle en C₁-C₄, alcoxy en C₁-C₄ ou -O-(CH₂)ₙ-COOH, -(CH₂)ₙ-COOH ou -NH-CO-(CH₂)ₙ-COOH, les radicaux pouvant être identiques ou différents, mais au moins un radical représentant -O-(CH₂)ₙ-COOH, -(CH₂)ₙ-COOH ou -NH-CO-(CH₂)ₙ-COOH, n étant un nombre entier de 1 à 6, et
R¹ représente un atome d'hydrogène ou un radical alcoxy-(C₁-C₆)-aryle(C₆-C₁₂),
avec un réactif de silylation, dans un solvant approprié à cet effet, et le composé silylé est ensuite converti, à l'aide d'un dérivé d'acide chloroformique, en composés de formule III 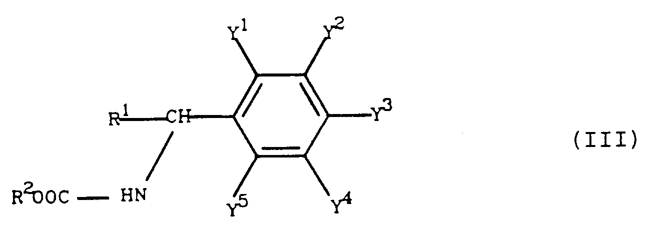 dans laquelle
R¹, Y¹, Y², Y³, Y⁴ et Y⁵ sont tels que définis ci-dessus, et
R² représente un radical aryle en C₆-C₁₂ qui est substitué par des substituants électroattracteurs, de préférence le groupe nitro et les halogènes,
on fait réagir les composés de formule III ainsi obtenus avec un hydrazide d'aminoacide de formule IV
R³-X-CO-NH-NH-R⁴ (IV)
dans laquelle
X représente un aminoacide ou iminoacide naturel ou non et est tel que défini plus haut,
R³ représente un groupe protecteur labile en présence de bases ou labile en présence d'acides faibles ou labile à l'hydrogénation, et
R⁴ représente un groupe alkyle en C₁-C₈, cycloalkyle en C₃-C₉, aryle en C₆-C₁₂, aryl(C₆-C₁₂)-alkyle(C₁-C₈), hétéroaryle ou hétéroaryl-alkyle(C₁-C₈) ou un atome d'hydrogène,
dans un solvant approprié, pour aboutir aux composés de formule V 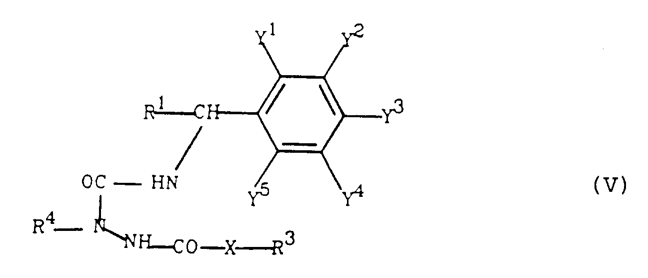 dans laquelle R¹, R³, R⁴ et Y¹, Y², Y³, Y⁴ et Y⁵ ont les significations données plus haut et, lorsque R³ représente un groupe protecteur labile à l'hydrogénation, on élimine ce groupe protecteur par hydrogénation sur un catalyseur palladié et, avant la réaction suivante, on le remplace par un groupe protecteur uréthane labile en présence de bases ou d'acides faibles,
le composé de formule V, dans lequel R¹, R⁴, Y¹, Y², Y³, Y⁴ et Y⁵ ont les significations données plus haut et R³ représente un groupe protecteur uréthanne labile en présence de bases ou d'acides faibles, est ensuite couplé à une résine par l'intermédiaire du groupement -O-(CH₂)ₙ-COOH, -(CH₂)ₙ-COOH ou -NH-CO-(CH₂)ₙ-COOH, à l'aide des réactifs de couplage usuels dans la chimie des peptides, on élimine le groupe protecteur R³, on effectue graduellement le couplage d'amino-, imino- ou aza-aminoacides naturels ou non, temporairement protégés par des groupes protecteurs de fonction amino labiles en présence de bases ou labiles en présence d'acides faibles, et éventuellement sous forme de leurs dérivés activés, et, une fois la synthèse terminée, on sépare les peptides de formule I de la résine par traitement par un acide de force moyenne, en éliminant à nouveau en même temps, ou à la suite de cela par des moyens appropriés, des groupes protecteurs de chaînes latérales temporairement introduits.

3. Procédé selon la revendication 1 ou 2, caractérisé en ce que l'on prépare le peptide Ac-D-Nal(2)-p-Cl-D-Phe-D-Trp-Ser-Tyr-D-Ser(α-L-Rha)-Leu-Arg-Pro-Azagly-NH₂.

4. Procédé selon la revendication 1 ou 2, caractérisé en ce que l'on prépare le peptide pGlu-His-Trp-Ser-Tyr-D-Ser(tBu)-Leu-Arg-Pro-Azagly-NH₂.
